# EUROPÄISCHE PATENTSCHRIFT

(11) **EP 1 485 215 B1**
(45) Veröffentlichungstag und Bekanntmachung des Hinweises auf die Patenterteilung: **18.01.2006**
(21) Anmeldenummer: 03707889.6
(22) Anmeldetag: 12.03.2003
(51) Int. Cl.: B08B 9/02, A61L 2/07

(54) **EINRICHTUNG ZUM DÄMPFEN VON DRUCKSTÖSSEN VON IN EINER FLÜSSIGKEITSLEITUNG STRÖMENDEN FLÜSSIGKEITEN**
METHOD AND DEVICE FOR ATTENUATING PRESSURE SURGES OF LIQUIDS FLOWING INSIDE A LIQUID CONDUIT
DISPOSITIF POUR ATTENUER LES COUPS DE BELIER DANS DES LIQUIDES S'ECOULANT DANS UNE CONDUITE DE LIQUIDE

(30) Priorität: 15.03.2002 AT 3992002
(43) Veröffentlichungstag der Anmeldung: 15.12.2004
(73) Patentinhaber: Sauer, Klaus, 66509 Rieschweiler-Mühlbach (DE); Moser, Gilbert, 8055 Graz (AT); Moser, Volker, 8010 Graz (AT)
(72) Erfinder: Sauer, Klaus, 66509 Rieschweiler-Mühlbach (DE); Moser, Gilbert, 8055 Graz (AT); Moser, Volker, 8010 Graz (AT)
(74) Vertreter: Kopecky, Helmut
(86) Internationale Anmeldenummer: PCT/AT2003/000072
(87) Internationale Veröffentlichungsnummer: WO 2003/078083

(56) Entgegenhaltungen:
- EP-A- 0 102 215
- EP-A- 0 107 459
- DE-A- 19 706 578
- US-A- 2 712 831
- US-A- 2 997 049
- PATENT ABSTRACTS OF JAPAN Bd. 0082, Nr. 62 (M-341), 30. November 1984 (1984-11-30) & JP 59 133900 A (MITSUI ZOSEN KK), 1. August 1984 (1984-08-01)

## Beschreibung

Die Erfindung betrifft eine Einrichtung zum Dämpfen von Druckstaßen von in einer Flüssigkeitsleitung strömenden Flüssigkeiten gemäß dem Oberbegriff des Anspruches 1.

Beim Transportieren von Flüssigkeiten durch Flüssigkeitsleitungen, insbesondere zum Zweck des Abfüllens in Behältnisse, müssen die Flüssigkeiten durch Steuer- und/oder Regelungsventile in ihrem Fluß auf bestimmte Durchflußwerte gebracht werden bzw. muß der Fluß der Flüssigkeiten unterbrochen werden, z.B. nach Erreichen eines vorbestimmten Füllstandes im Behältnis. Es kommt daher in den Flüssigkeitsleitungen andauernd zu mehr oder weniger großen Druckschwankungen. Diese können sehr erheblich sein und bei großen Abfüllanlagen, beispielsweise beim Abfüllen von Bier, Milch, etc., eine pulsierende Charakteristik aufweisen.

Druckstöße können Maximaldrücke von über dem Zweieinhalbfachen des normalen Flüssigkeitsleitungsdruckes ergeben. Hierdurch werden die Armaturen und sonstigen Apparate, wie z.B. Membranfilter, Plattenwärmetauscher sowie vor allem auch Dichtungen überbeansprucht. Bei höheren statischen Leitungsdrücken sind solche Druckstöße entsprechend der Dauerfestigkeitshypothese als noch kritischer einzustufen.

Zur Abhilfe ist es bekannt, Windkessel zur Dämpfung der Druckstöße einzusetzen. Solche Windkessel sind zwar eine bewährte und einfache Dämpfungseinrichtung, jedoch bedingen unterschiedliche Drücke in den Flüssigkeitsleitungen unterschiedlich große Gasräume im Windkessel mit unterschiedlichen Eigenfrequenzen, was für nachgeschaltete elektronische Regelungen Probleme ergeben kann. Ein weiterer großer Nachteil solcher Windkessel ist, daß eine Reinigung während eines Betriebszyklus nicht möglich ist. Eine Reinigung eines Windkessels bedingt eine Absperrung desselben von der Rohrleitung, wobei nach der Reinigung die Gefahr des Vorhandenseins von Resten des Reinigungsmittels nicht ausgeschlossen werden kann. Eine Komplettspülung des Windkessels ist nur zeitraubend durchführbar.

Man hat auch versucht, mit elektronischen Methoden Druckstöße zu dämpfen, d.h. elektronische Regel- und Dämpfungseinnchtungen vorzusehen. Diese sind jedoch von der Schnelligkeit der Systemsensorik und -aktorik abhängig. Es kann trotzdem vor allem bei hochfrequenten Schwingungen zu Resonanzen kommen, die das Problem der Druckstöße oft noch verschärfen. Anderseits kann auch eine Überdämpfung des Systems zu nicht mehr ausgleichbaren Druckschwankungen führen. Ein besonderes Problem stellt hierbei das Auftreten von stark unterschiedlichen Frequenzen der Druckstöße dar.

Aus der Gebrauchsmusterschrift AT 001740 U1 ist eine Nebenschlussrohrleitung, d.h. eine Bypass-Leitung, zu einer Flüssigkeitsleitung, wie einer Bierleitung, bekannt, die im Normalzustand mit Gas gefüllt ist und zur Aufnahme von Druckstößen dient. Bei dieser bekannten Einrichtung können jedoch Druckstöße durch die Bypass-Leitung durchschlagen. Weiters ist eine Reinigung, insbesondere mit einer Sterilisationsflüssigkeit, umständlich durchzurühren, da einerseits die Bypass-Leitung und andererseits die Flüssigkeitsleitung getrennt zu behandeln sind, und weiters kommt es bei einem Produktwechsel zu Schwierigkeiten infolge einer unzulässigen Vermischung der nacheinander durch die Flüssigkeitsleitung strömenden unterschiedlichen Produkte.

Aus der DE 197 06 578 C2 ist eine Einrichtung zum Dämpfen von Druckstößen von in einer Flüssigkeitsleitung strömenden Flüssigkeiten gemäß dem Oberbegriff des Anspruches 1 bekannt. Gemäß dieser Einrichtung gelingt es im Bypass einen Gaspolster auszubilden, der Druckschwankungen, die in der Flüssigkeitsleitung auftreten, ausgleichen kann. Der Bypass bildet hierbei einen einzigen Gasraum zur Pufferung der Druckstöße

Die Erfindung bezweckt die Vermeidung dieser Nachteile und Schwierigkeiten und stellt sich die Aufgabe, eine Einrichtung der eingangs beschriebenen Art zu schaffen, welche nicht nur eine hervorragende Dämpfung von Druckstößen ermöglicht, sondern auch eine zuverlässige Reinigung der Einrichtung ermöglicht, u.zw, mit einfachen Mitteln, sodaß die Anwendung auch für biologisch gefährdete Produkte, wie z.B. Lebensmittelprodukte, ohne einen großen Aufwand zu erfordern, gegeben ist. Zudem soll auch ein Produktwechsel ohne wesentliche Mischung der unterschiedlichen Produkte durchführbar sein.

Diese Aufgabe wird erfindungsgemäß durch die im kennzeichnenden Teil des Anspruches 1 enthaltenen Merkmale gelöst.

Hierdurch gelingt es, ein Überströmen des Bypasses durch Flüssigkeit mit Sicherheit zu verhindern, d.h. die Gaspolster im Bypass bei jeder Art von Störung aufrecht zu erhelten. Zudem wird ein gegebenenfalls vorgesehenes Regelventil geschont. Ein aus dem Bypass entweichender und in die Flüssigkeitsleitung gelangender Gaspolster würde z.B. eine Filteraktivität eines Filters, das zum Filtern der Flüssigkeit vorgesehen ist, verringern, und es würde eine undefinierbare Gasmenge abgehen.

Vorzugsweise ist jeweils benachbart zu die Bypass-Leitung von der Flüssigkeitsleitung trennenden Absperrventilen und innerhalb der von den Absperrventilen gebildeten Abschnitte in der Bypass-Leitung jeweils ein Entleerhahn vorgesehen.

Hierdurch gelingt es, einen Produktwechsel ohne Reinigung exakt durchzuführen. Ein vom bisher strömenden Produkt unterschiedliches Produkt gelangt bis zur Füllstation, wird dort gestoppt, worauf die Absperrventile der Bypass-Leitung geschlossen werden und die Entleerhähne der Bypass-Leitung so lange geöffnet werden, bis das erste Produkt völlig abgelassen ist.

Vorteilhaft weist das in der Füssigkeitsleitung vorgesehene Absperrventil im geschlossenen Zustand eine einen geringen Durchfluß ermöglichende Leckageöffnung auf und sind zweckmäßig das in der Flüssigkeitsleitung und in der Bypass-Leitung vorgesehene Absperrventil mittels einer Steuereinheit alternativ betätigbar.

Als Reinigungsmedium ist vorzugsweise eine Sterilisationsflüssigkeit vorgesehen. Die erfindungsgemäße Einrichtung ist in besonders hohem Maße für biologisch gefährdete Produkte, insbesondere Lebensmittelprodukte, einsetzbar.

Zur Sicherung eines Flüssigkeitsniveaus, d.h. zwecks Verhinderns des Ansteigens desselben über ein vorbestimmtes Ausmaß, sind vorzugsweise in der Bypass-Leitung im vorbestimmten Abstand vom ersten und vom zweiten Absperrventil Sonden zum Feststellen eines Flüssigkeitsstandes vorgesehen wobei zweckmäßig eine Steuerungseinheit vorgesehen ist, die mit mindestens einer der Gaszuführungsleitungen zur Bypass-Leitung gekoppelt ist, sodaß bei Anzeigen eines Flüssigkeitsstandes eine automatische Gasspülung und damit ein Abführen von Flüssigkeit aus der Bypass-Leitung stattfindet.

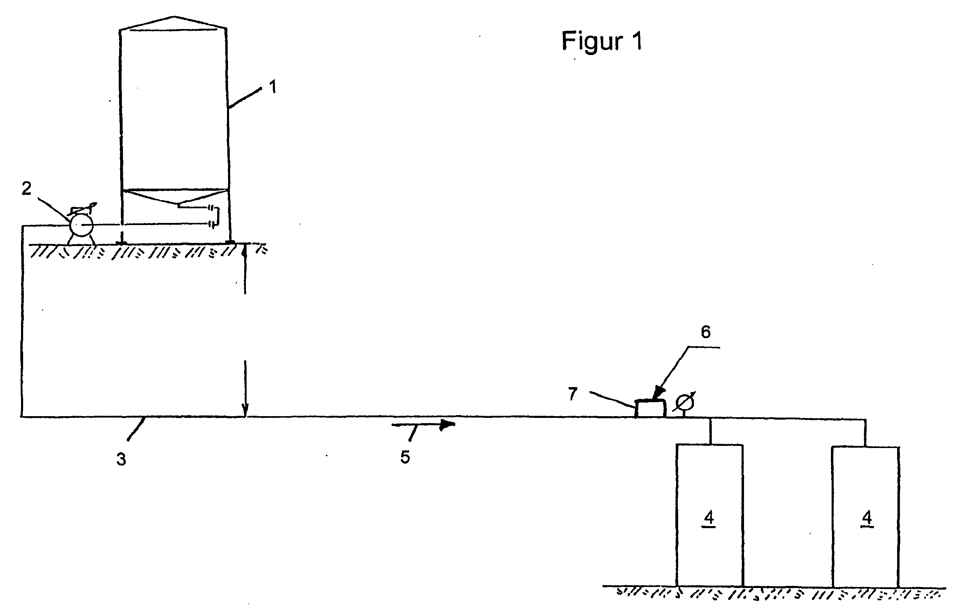

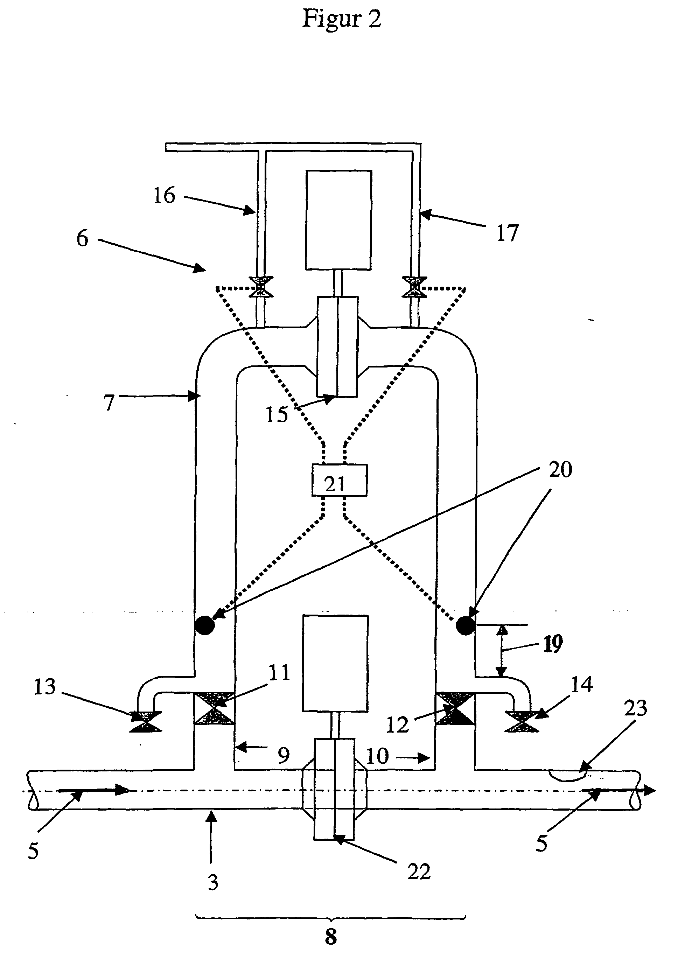

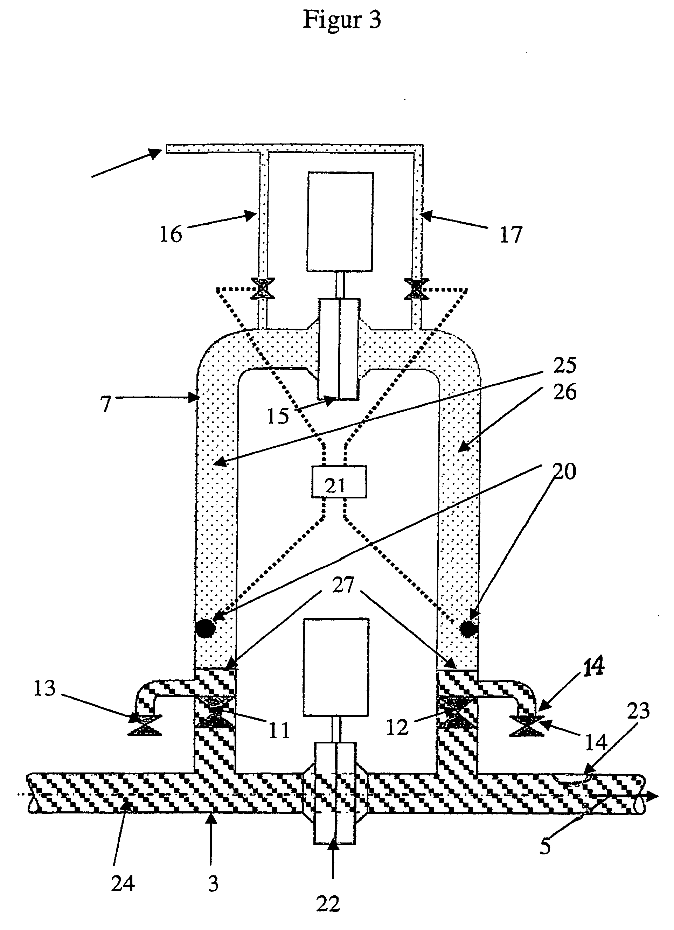

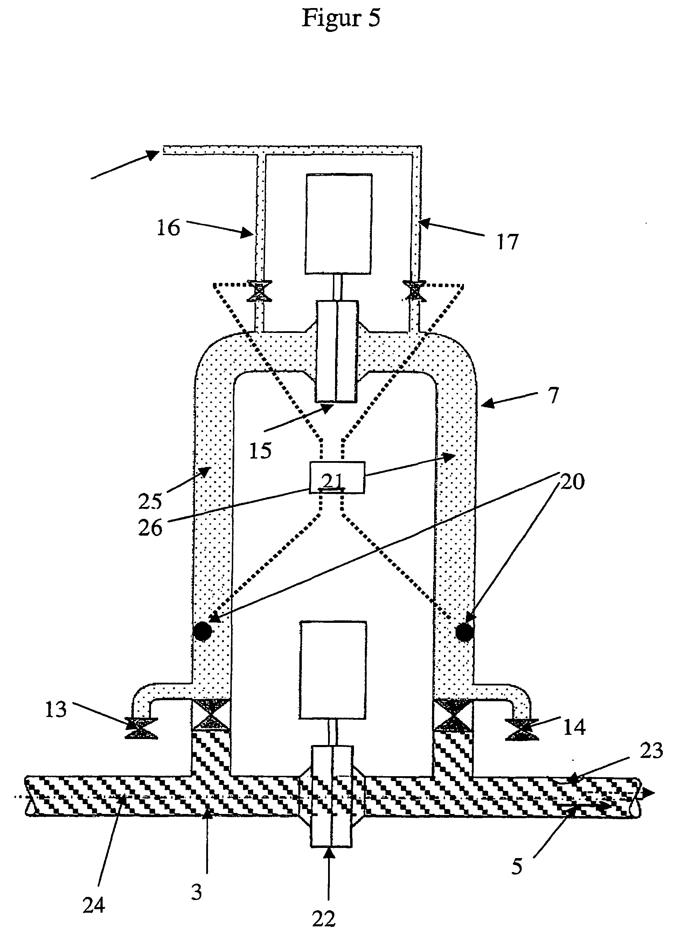

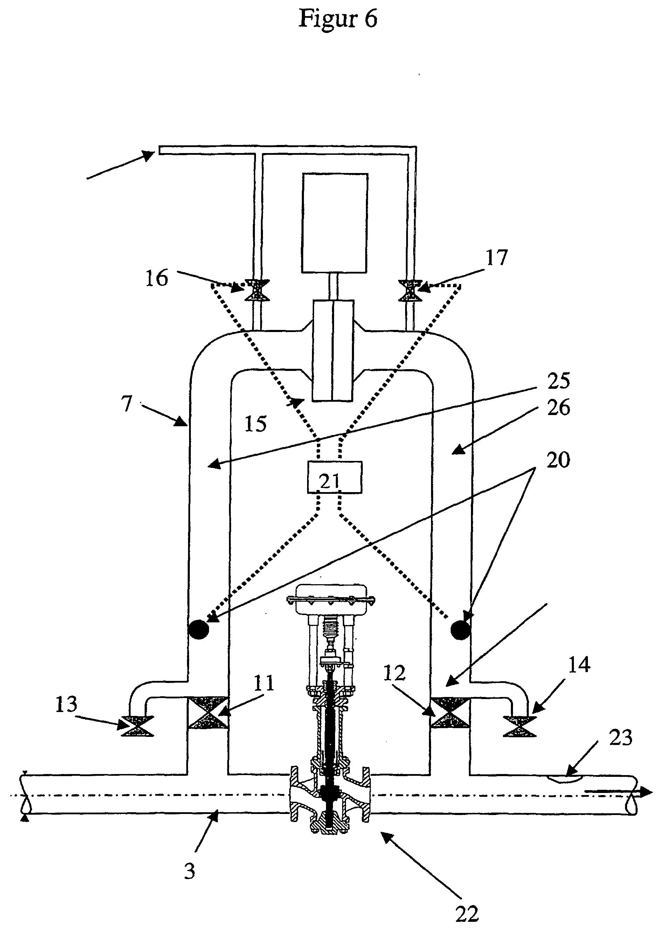

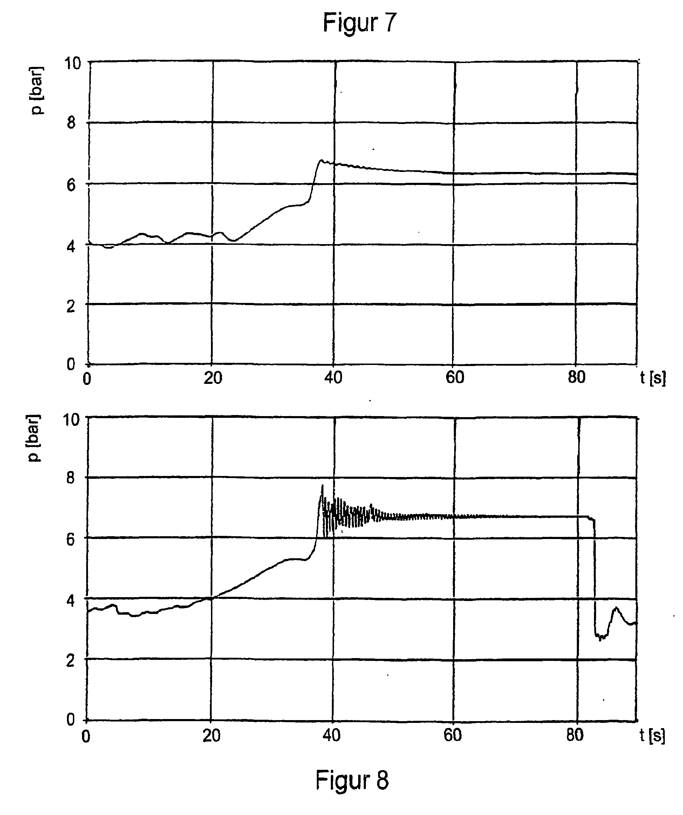

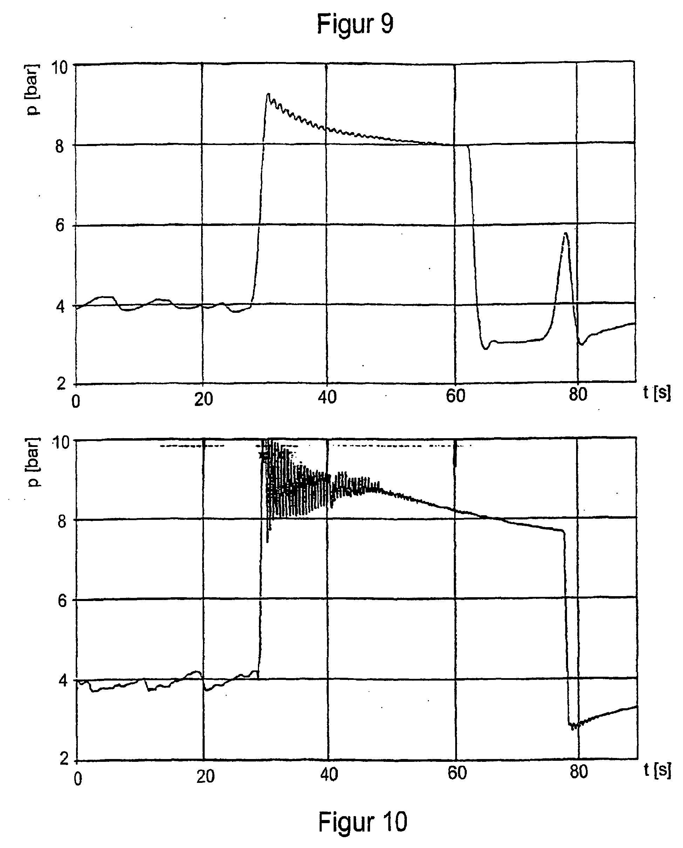

Die Erfindung ist nachfolgend anhand der Zeichnung, in der ein Ausführungsbeispiel dargestellt ist, näher erläutert. Fig. 1 zeigt eine Abfüllanlage für Frischmilch in schematischer Darstellung. Fig. 2 gibt die erfindungsgemäße Einrichtung im Detail wieder. Die Fig. 3 bis 5 veranschaulichen verschiedene Betriebszustände der erfindungsgemäßen Einrichtung. Fig. 6 gibt eine Variante dieser Einrichtung wieder. In den Fig. 7 bis 10 sind in der Flüssigkeitsleitung auftrerende Druckstöße in Diagrammform veranschaulicht, u. zw. in den Fig. 7 und 9 mit erfindungsgemäßer Einrichtung und in den Fig. 8 und 10 ohne erfindungsgemäße Einrichtung.

Gemäß der in Fig. 1 dargestellten Abfüllanlage für eine Flüssigkeit, hier für Frischmilch, gelangt Frischmilch von einem Vorratstank 1 über eine Zuführpumpe 2 und eine Flüssigkeitsleitung 3 zu zwei Abfüllmaschinen 4, in denen taktweise Milchflaschen oder sonstige Behältnisse, wie laminierte Kartons etc., befüllt werden. Zwischen dem Frischmilchtank und der ersten in Strömungsrichtung 5 angeordneten Abfüllanlage ist eine erfindungsgemäße Einrichtung 6 vorgesehen, die in Fig. 1 nur schematisch angedeutet ist, die jedoch in Fig. 2 im Detail dargestellt ist. Diese Einrichtung 6 ist zweckmäßig knapp vor den Abfüllmaschine 4 in die Flüssigkeitsleitung eingebaut.

Gemäß der in Fig. 2 dargestellten Ausführungsform weist die erfindungsgemäße Einrichtung 6 eine Bypass-Leitung 7 auf, die einen vorbestimmten Abschnitt 8 der Flüssigkeitsleitung 3 überbrückt. Im Bereich der Einmündungen 9 und 10 der Bypass-Leitung 7 in die Flüssigkeitsleitung 3 ist die Bypass-Leitung 7 mit einem ersten und einem zweiten Absperrventil 11, 12 versehen, sodaß es möglich ist, die Bypass-Leitung 7 leitungsmäßig komplett von der Flüssigkeitsleitung zu trennen.

Benachbart zum ersten und zum zweiten Absperrventil 11, 12 sind in der Bypass-Leitung jeweils ein absperrbarer Entleerhahn 13, 14 vorgesehen, u.zw. innerhalb des von den ersten und zweiten Absperrventilen 11, 12 gebildeten Abschnitts der Bypass-Leitung 7. Ein drittes Absperrventil 15 befindet sich in der Bypass-Leitung 7 in etwa dem gleichen Abstand von den Mündungen der Bypass-Leitung 7 (bei 9 bzw. 10) in die Flüssigkeitsleitung 3.

In Strömungsrichtung 5 der Flüssigkeit vor und nach dem dritten Absperrventil 15 sind in die Bypass-Leitung 7 einmündende absperrbare Gaszuführungsleitungen 16, 17 vorgesehen.

Zur Vermeidung eines zu hohen Flüssigkeitsniveaus in der Bypass-Leitung 7 sind in dieser im vorbestimmten Abstand 19 des ersten und zweiten Absperrventils Sonden 20 vorgesehen, mit denen das Vorhandensein von Flüssigkeit in dem Niveau der Sonden 20 festgestellt werden kann. Werden diese Sonden 20 von Flüssigkeit überspült, geben diese Sonden 20 ein Signal ab, welches über eine Steuereinheit 21 den Zutritt eines Gases über die Gaszuführungsleitungen 16 oder 17 in den entsprechenden überfluteten Abschnitt der Bypass-Leitung 7 steuern.

In der Flüssigkeitsleitung 3 ist in dem Abschnitt 8, der von der Bypass-Leitung 7 überbrückt ist, ein viertes Absperrventil 22 eingebaut, welches den Hauptstrom, d.h. den Flüssigkeitsstrom der über die Flüssigkeitsleitung 3 zu den Abfüllmaschinen 4 oder dgl. geführt wird, absperrt. Dieses Ventil 22 ist entweder als Absperrventil (Fig.2) oder als Regelventil, wie in Fig. 6 dargestellt, ausgebildet. Bei einer Ausbildung als Absperrventil, z.B. verwirklicht als Hahnschieber, ist der Schieber vorzugsweise mit einem Loch versehen, wobei dieses Loch beispielsweise einen Durchmesser von einigen wenigen Millimetern bis 20 mm aufweist. Hierdurch wird ein geringes Weiterströmen des in der Flüssigkeitsleitung strömenden Mediums gewährleistet, auch wenn das Ventil geschlossen ist. Dieses Loch bildet somit eine den Durchfluß freilassende Leckageöffnung. In der Flüssigkeitsleitung ist weiters in Strömungsrichtung nach der Bypass-Leitung 7 eine Drossel 23 eingebaut.

Die Funktion der erfindungsgemäßen Einrichtung 6 ist folgende:

Während des normalen Förderns von Flüssigkeit 24 (Fig. 3) über die Flüssigkeitsleitung 3 sind die ersten und zweiten Absperrventile 11, 12 geöffnet und die Entleethähne 13, 14 geschlossen. In der Bypass-Leitung 7, in der das dritte Absperrventil 15 geschlossen ist, befinden sich in den beiden durch das geschlossene dritte Ventil gebildeten Räumen 25, 26 Gaspolster, u. zw. in der Art und Weise, daß das Flüssigkeitsniveau 27 unter den in diesen Räumen 25, 26 vorgesehenen Sonden 20 liegt. Während des Förderns der Flüssigkeit 24 ist das vierte Absperrventil 22 geöffnet. Bei Auftreten von Druckstößen oder hochfrequenten Druckschwankungen werden diese von in den beiden gasgefüllten Räumen 25, 26 der Bypass-Leitung 7 vorhandenen Gaspolstern aufgenommen bzw. abgebaut.

Zum Erzeugen eines Gaspolsters wird vorzugsweise CO₂ oder Sterilluft verwendet.

Der Gasdruck der Gaspolster ist an den herrschenden statischen Betriebsdruck der Flüssigkeit derart angepaßt, daß das Flüssigkeitsniveau 27 stets unter den Sonden 20 verbleibt. Dieser Zustand ist in Fig. 3 dargestellt.

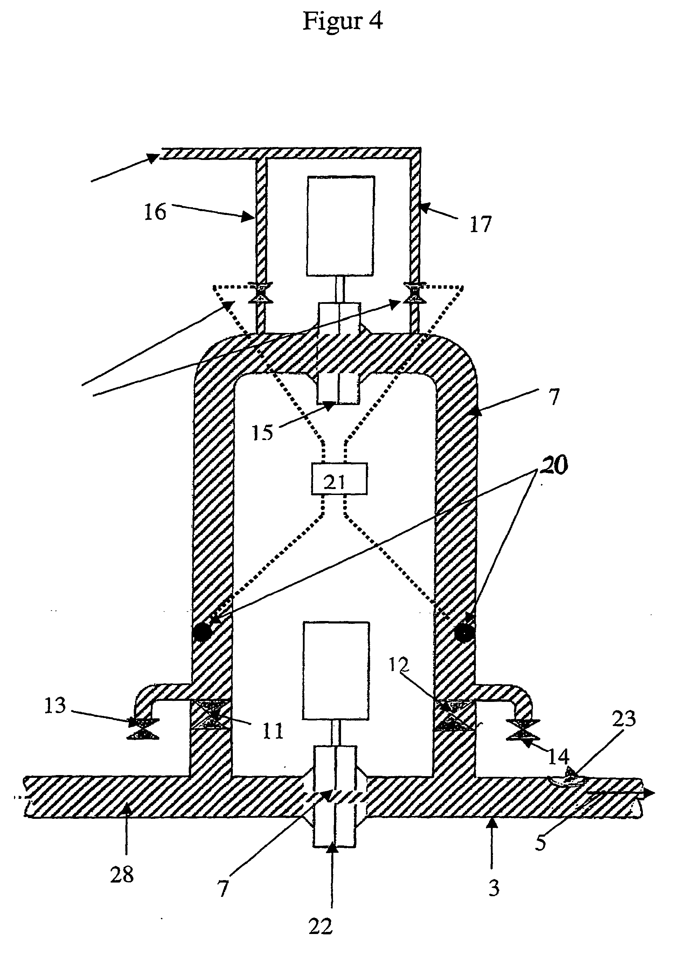

Zum Zweck der Reinigung (Fig. 4) der Flüssigkeitsleitung 3 bleiben die ersten und zweiten Absperrventile 11, 12 geöffnet es werden jedoch das dritte Absperrventil 15 geöffnet und das vierte Absperrventil 22 geschlossen. Die beiden Entleerhähne 13. 14 werden kurzzeitig geöffnet bzw. getaktet, um aus den Rohrleitungsteilen, die von der Bypass-Leitung 7 zu den Entleerhähnen 13, 14 führen, ebenfalls die zuvor geförderte Flüssigkeit 24 zu entfernen und diese Rohrleitungen mit einem Reinigungsmedium 28 zu füllen und zu durchströmen. Die Gaszuführungsleitungen werden ebenfalls kurzzeitig geöffnet oder getaktet (vgl. Fig. 4).

Infolge der Leckageöffnung des Absperrventiles 22 bzw. eines nicht zur Gänze geschlossenen Absperrventiles nach Fig. 6 wird sowohl die Flüssigkeitsleitung 3 als auch die Bypass-Leitung 7 vom Reinigungsmedium durchströmt. Die Drossel 23 sorgt für einen Druckstoßabfang bei Strömen der Flüssigkeit oder nachfolgend fließendem Wasser.

Anstelle eines gleichzeitigen Durchströmens der Flüssigkeitsleitung 3 und der Bypass-Leitung 7 ist es auch möglich, während des Reinigungszyklus die Absperrventile 15 und 22 alternativ pulsierend zu öffnen und zu schließen, so daß das Reinigungsmedium die Leitungen 3 und 7 stoßweise pulsierend durchströmt.

Um einen Produktwechsel durchführen zu können, d.h. z.B. die Reinigungsflüssigkeit vollständig zu entfernen, werden zunächst die ersten und zweiten Absperrventile 11, 12 geschlossen, die Entleerhähne 13, 14 geöffnet und der Abschnitt zwischen den ersten und den zweiten Absperrventilen 11, 12 der Bypass-Leitung 7 mit Gas geflutet. Hierbei ist zweckmäßig das dritte Absperrventil 15 geschlossen. Das vierte Absperrventil 22 ist geöffnet, sodaß die nunmehr nach der Reinigungsflüssigkeit durch die Flussigkeitsleitung neu einströmende Flüssigkeit diese verdrängt. Nach Entfernung der Reinigungsflüssigkeit werden das erste und das zweite Absperrventil 11, 12 geöffnet und die Entleerhähne 13, 14 geschlossen, sodaß wiederum der in Fig. 3 dargestellte Normalzustand während des Förderns der Flüssigkeit über die Flüssigkeitsleitung 3 hergestellt ist. Analog wird beim Übergang des Förderns einer Produktflüssigkeit zu einer anderen Produktflüssigkeit vorgegangen.

Aus den Diagrammen der Fig. 7 bis 10 ist zu erkennen, in welchem Ausmaß Druckstöße mit der erfindungsgemäßen Einrichtung gedämpft werden können. So zeigt Fig. 7 beispielsweise einen gedämpften Druckstoß bei Normalstop der Flüssigkeitsförderung d.h. mit elektronischer Dämpfung und Fig. 8 den Druckverlauf ebenfalls bei Normalstop, jedoch ohne erfindungsgemäße Einrichtung 6. Es ist nicht nur ein deutlich höherer Maximaldruck zu erkennen, sondern auch eine hohe Frequenz der nur langsam abklingenden Druckschwingungen, die zu einer enormen Belastung der gesamten Anlage führen.

Die Fig. 9 und 10 zeigen ähnliche Diagramme für einen Schnell- bzw. Notstop der zu fördernden Flüssigkeit, u. zw. Fig. 9 mit erfindungsgemäßer Einrichtung 6 und Fig. 10 ohne erfindungsgemäße Einrichtung 6.

Die Erfindung beschränkt sich nicht auf die in der Zeichnung dargestellten Ausführungsbeispiele, sondern kann in verschiedener Hinsicht modifiziert werden. So wird als Bypass-Leitung jedwede von einer Stelle der Flüssigkeitsleitung 3 ausgehende und an anderer Stelle der Flüssigkeitsleitung 3 einmündende Rohrleitung oder gegebenenfalls auch ein Rohrleitungssystem mit gleicher Funktion verstanden. Desgleichen kann auch die Flüssigkeitsleitung 3 als Rohrleitungssystem ausgebildet sein.

## Patentansprüche

1. Einrichtung (6) zum Dämpfen von Druckstößen von in einer Flüssigkeitsleitung (3) strömenden Flüssigkeiten (24), z.B. einer biologisch gefährdeten Flüssigkeit, sowie zum Reinigen, insbesondere zum Sterilisieren, der Flüssigkeitsleitung (3) mit einer einen Abschnitt (8) der Flüssigkeitsleitung (3) überbrückenden Bypass-Leitung (7), wobei ein Absperrventil (22) in der Flüssigkeitsleitung (3) zwischen den Einmündungen (9, 10) der Bypass-Leitung (7) sowie eine in die Bypass-Leitung (7) einmündende absperrbare Gaszuführungsleitung (16, 17) vorgesehen sind, **dadurch gekennzeichnet, daß** die Bypass-Leitung (7) wahlweise in zwei gasgefüllte Räume (25, 26), in die jeweils eine eigene Gaszuführungsleitung (16, 17) mündet, vorzugsweise zwei etwa volumsgleiche gasgefüllte Räume (25, 26), mittels eines Absperrventils (15) teilbar ist, die beide mit der Flüssigkeitsleitung (3) zwecks Dämpfens von Druckstößen kommunizieren.

2. Einrichtung nach Anspruch 1, **dadurch gekennzeichnet, daß** jeweils benachbart zu die Bypass-Leitung (7) von der Flüssigkeitsleitung (3) trennenden Absperrventilen (11 bzw. 12) und innerhalb der von den Absperrventilen (11, 12) gebildeten Abschnitte in der Bypass-Leitung (7) jeweils ein Entleerhahn (13, 14) vorgesehen ist.

3. Einrichtung nach Anspruch 1 oder 2, **dadurch gekennzeichnet, daß** das in der Füssigkeirsleitung (3) vorgesehene Absperrventil (22) im geschlossenen Zustand eine einen geringen Durchfluß ermöglichende Leckageöffnung aufweist.

4. Einrichtung nach einem oder mehreren der Ansprüche 1 bis 3, **dadurch gekennzeichnet, daß** das die Bypass-Leitung (7) teilende und das in der Flüssigkeitsleitung (3) vorgesehene Absperrventil (15, 22) mittels einer Steuereinheit alternativ betätigbar ist.

5. Einrichtung nach einem oder mehreren der Ansprüche 1 bis 4. **dadurch gekennzeichnet, daß** in der Bypass-Leitung (7) im vorbestimmten Abstand vom ersten und vom zweiten Absperrventil (11, 12) an sich bekannte Sonden (20) zum Feststellen eines Flüssigkeitsstandes vorgesehen sind

6. Einrichtung nach Anspruch 5, **dadurch gekennzeichnet, daß** eine Steuerungseinheit (21) vorgesehen ist, die mit mindestens einer der Gaszuführungsleitungen (16, 17) zur Bypass-Leitung (7) gekoppelt ist, sodaß bei Anzeigen eines Flüssigkeitsstandes eine automatische Gasspülung und damit ein Abführen von Flüssigkeit aus der Bypass-Leitung (7) stattfindet.

## Claims

1. A device (6) for attenuating pressure surges of liquids (24), e.g. of a biologically vulnerable liquid, flowing inside a liquid conduit (3) and for cleaning, in particular sterilizing, the liquid conduit (3) comprising a bypass conduit (7) that bridges over a section (8) of the liquid conduit (3), wherein an isolating valve (22) in the liquid conduit (3) between the outlets (9, 10) of the bypass conduit (7) as well as a lockable gas supply conduit (16, 17) running into the bypass conduit (7) are provided, **characterized in that** the bypass conduit (7) is optionally divisible into two gas-filled spaces (25, 26) into each of which a separate gas supply conduit (16, 17) runs, preferably two gas-filled spaces (25, 26) of roughly equal volumes, by means of an isolating valve (15), which both communicate with the liquid conduit (3) for the purpose of attenuating pressure surges.

2. A device according to claim 1, **characterized in that** a pet cock (13, 14) is in each case provided adjacent to isolating valves (11 and 12, respectively) separating the bypass conduit (7) from the liquid conduit (3) and within the sections in the bypass conduit (7) that are formed by the isolating valves (11, 12).

3. A device according to claim 1 or 2, **characterized in that** the isolating valve (22) provided in the liquid conduit (3) has, in the closed state, a leakage opening enabling a small flow.

4. A device according to one or several of claims 1 to 3, **characterized in that** the isolating valve (15, 22) dividing the bypass duct (7) and provided in the liquid conduit (3) is alternatively operable by means of a control unit.

5. A device according to one or several of claims 1 to 4, **characterized in that** probes (20) known per se for determining a liquid level are provided in the bypass conduit (7) at a predetermined distance from the first and the second isolating valves (11, 12).

6. A device according to claim 5, **characterized in that** a control unit (21) is provided which is coupled to at least one of the gas supply conduits (16, 17) leading to the bypass conduit (7) so that, if a liquid level is indicated, an automatic gas flushing and hence a discharge of liquid from the bypass conduit (7) will take place.

## Revendications

1. Dispositif (6) destiné à atténuer les coups de bélier de liquides (24) circulant dans une conduite de liquide (3), par exemple d'un liquide biologiquement à risque, ainsi qu'à nettoyer, notamment à stériliser, la conduite de liquide (3) avec une conduite de dérivation (7) surmontant une section (8) de la conduite de liquide (3), une soupape d'arrêt (22) dans la conduite de liquide (3) entre les embouchures (9, 10) de la conduite de dérivation (7) ainsi qu'une conduite d'amenée de gaz (16, 17) pouvant être fermée, entrant dans la conduite de dérivation (7) étant prévues, **caractérisé en ce que** la conduite de dérivation (7) peut être divisée au choix en deux chambres remplies de gaz (25, 26), dans lesquelles débouche respectivement une conduite d'amenée de gaz (16, 17) propre, de préférence en deux chambres remplies de gaz (25, 26) présentant approximativement le même volume, au moyen d'une soupape d'arrêt (15), lesquelles chambres communiquent toutes les deux avec la conduite de liquide (3) afin d'atténuer les coups de bélier.

2. Dispositif selon la revendication 1, **caractérisé en ce qu'**un robinet de purge (13, 14) est prévu respectivement au voisinage des soupapes d'arrêt (11 et/ou 12) séparant la conduite de dérivation (7) de la conduite de liquide (3) et à l'intérieur des sections formées par les soupapes d'arrêt (11, 12) dans la conduite de dérivation (7).

3. Dispositif selon la revendication 1 ou 2, **caractérisé en ce que** la soupape d'arrêt (22) prévue dans la conduite de liquide (3) comprend à l'état fermé un orifice de fuite permettant un faible écoulement.

4. Dispositif selon l'une ou plusieurs des revendications 1 à 3, **caractérisé en ce que** la soupape d'arrêt (15, 22) séparant la conduite de dérivation (7) et prévue dans la conduite de liquide (3) peut être actionnée en variante au moyen d'une unité de commande.

5. Dispositif selon l'une quelconque des revendications 1 à 4, **caractérisé en ce que** des sondes (20) connues en soi, destinées à déterminer un niveau de liquide, sont prévues dans la conduite de dérivation (7) à distance prédéfinie des première et seconde soupapes d'arrêt (11, 12).

6. Dispositif selon la revendication 5, **caractérisé en ce qu'**une unité de commande (21) est prévue, qui est couplée à au moins une des conduites d'amenée de gaz (16, 17) en direction de la conduite de dérivation (7), de sorte qu'en affichant un niveau de liquide, il se produit un barbotage automatique et de ce fait une évacuation du liquide hors de la conduite de dérivation (7).
